# EUROPÄISCHE PATENTANMELDUNG

(11) **EP 4 599 827 A1**
(43) Veröffentlichungstag der Anmeldung: **13.08.2025**
(21) Anmeldenummer: 24156855.9
(22) Anmeldetag: 09.02.2024
(51) Int. Cl.: A61K 31/198, A61K 8/00, A61P 9/08, A61P 17/02, A61P 17/00, A61K 31/045, A61K 31/122, A61K 31/164, A61K 31/185, A61K 31/4188, A61K 31/455, A61K 31/505, A61K 31/522, A61K 31/573

(54) **DMG ZUR INDUKTION ENDOTHEL-ABGELEITETER NO-ABHÄNGIGER VASODILATATION**

(71) Anmelder: Dr. Kurt Wolff GmbH & Co. KG, 33611 Bielefeld (DE)
(72) Erfinder: ZUR WIESCHE SCHULZE, Erik, 33611 Bielefeld (DE); VÖLKER, Jörn Michael, 33611 Bielefeld (DE); NEUHAUS, Bernhard, 33611 Bielefeld (DE)
(74) Vertreter: Weickmann & Weickmann PartmbB

(57) **Zusammenfassung**

Die Anmeldung betrifft die Verwendung einer Zusammensetzung umfassend N,N-Dimethylglycin (DMG) und/oder ein Salz davon zur Induktion von Endothel-abgeleiteter NOabhängiger Vasodilatation.

## Beschreibung

Die Anmeldung betrifft die Verwendung einer Zusammensetzung umfassend N,N-Dimethylglycin (DMG) und/oder ein Salz davon zur Induktion von Endothel-abgeleiteter NO-abhängiger Vasodilatation.

Stickstoffmonoxid (NO) ist für seine erweiternde Wirkung auf die Blutgefäße bekannt. Protektive Wirkungen von NO und seiner Vorstufe L-Arginin sind bei Gesunden ebenso wie bei Patienten mit kardiovaskulären Erkrankungen wie Arteriosklerose, Bluthochdruck und Durchblutungsstörungen zwischenzeitlich gut belegt.

Die gefäßerweiternde Wirkung des Stickstoffmonoxids macht man sich bei der Behandlung einer Reihe von Erkrankungen wie beispielsweise Angina pectoris zunutze. Durch die Verwendung von NO-freisetzenden Substanzen wird dabei Vasodilatation bewirkt.

Die hierzu verwendeten Substanzen wie beispielsweise Nitroglycerin, Molsidomin oder Isosorbiddinitrat (ISDN) sind in ihrer Anwendung aber häufig durch mögliche Wechselwirkungen, Nebenwirkungen oder Kontraindikationen beschränkt, so dass immer ein großer Bedarf an neuen NO-induzierenden Ansätzen besteht.

Ein erster Aspekt der Erfindung betrifft die Verwendung einer Zusammensetzung umfassend N,N-Dimethylglycin (Dimethylglycin, DMG) und/oder ein Salz davon zur Induktion Endothel-abgeleiteter NO-abhängiger Vasodilatation bzw. eine Zusammensetzung umfassend N,N-Dimethylglycin (Dimethylglycin, DMG) und/oder ein Salz zur Induktion Endothel-abgeleiteter NO-abhängiger Vasodilatation.

Erfindungsgemäß hat sich nunmehr überraschend gezeigt, daß auch DMG Endothel-abgeleitete NO-abhängige Vasodilatation induzieren oder initiieren kann kann.

DMG kommt in Pflanzen, Tieren und dem Menschen vor, wobei es im Menschen nur in sehr geringen Mengen gebildet wird. Es entsteht bei einer mehrstufigen Biosynthese von Glycin aus Cholin als Zwischenprodukt durch Transaminierung von Betain mit Betain-Homocystein-Methylase. DMG und DMG-Na unterstützen nachweislich auch das Gewebe und die Zellenfunktionen, z. B. durch Verbesserung antioxidativer Kapazitäten und der Sauerstoffverwertung, Förderung der Geweberegeneration und Verbesserung der Immunantwort/Abwehr.

N,N-Dimethylglycin, auch (Dimethylamino)essigsäure, wird durch die folgende chemische Formel 1 dargestellt:

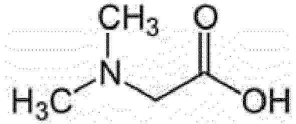

Erfindungsgemäß ist nicht nur der Einsatz von Dimethylglycin, sondern auch der von dessen Salzen, Solvaten und Hydraten. Dabei handelt es sich bevorzugt um pharmazeutisch bzw. kosmetisch annehmbare Salze von Dimethylglycin. Das Salz ist besonders bevorzugt ein wasserlösliches Salz mit einer Löslichkeit in Wasser von mindestens 10 g/l bei 20°C.

In einer bevorzugten Aufführungsform ist das Salz von Dimethylglycin ein Alkali-, Erdalkali- oder Ammoniumsalz von Dimethylglycin.

Beispiele sind Natrium-, Kalium-, Calcium-, Magnesium- und Ammoniumsalze. Bei den Ammoniumsalzen trägt das Ammoniumkation eine bis vier Alkylgruppen mit jeweils unabhängig voneinander 1 bis 4 Kohlenstoffatomen. Erfindungsgemäß bevorzugt sind das Natrium- und Kaliumsalz von Dimethylglycin, insbesondere das Natriumsalz von Dimethylglycin, nämlich Natrium-N,N-dimethylglycinat.

In einer alternativ bevorzugten Ausführungsform kann das Salz von Dimethylglycin das Salz einer anorganischen und/oder organischen Säure mit Dimethylglycin sein.

Beispiele für Salze von Dimethylglycin mit einer anorganischen Säure sind das Hydrochlorid, Hydrobromid, Hydroiodid, Hydrogensulfat, Sulfat, Hydrogensulfit, Sulfit, Hydrogencarbonat, Carbonat, Monophosphat, Diphosphat und Triphosphat von Dimethylglycin sowie Mischungen daraus. Insbesondere bevorzugt ist das Hydrochlorid von Dimethylglycin.

Erfindungsgemäß werden die beschriebenen Zusammensetzungen umfassend N,N-Dimethylglycin (DMG) und/oder ein Salz davon entsprechend zur Induktion von Endothel-abgeleiteter NO-abhängiger Vasodilatation bzw. Gefäßdilation eingesetzt. Ein Aspekt der Erfindung betrifft entsprechend eine Zusammensetzung umfassend N,N-Dimethylglycin (DMG) und/oder ein Salz davon zur Verwendung in der Behandlung und/oder Vorbeugung von Erkrankungen, welche NO-assoziiert sind, durch NO-Mangel bedingt sind und/oder durch NO-Induktion und/oder NO-Freisetzung unter Vasodilatation bzw. Gefäßdilation therapiert werden können.

Vasodilatation bezeichnet die Ausdehnung oder Erweiterung der Blutgefäße, mit anderen Worten die Vergrößerung ihres Lumens. Die Vasodilatation ist eine physiologische Reaktion, die dazu führt, dass der Gefäßquerschnitt und damit die Durchblutung hinter dem betroffenen Gefäßabschnitt vergrößert wird. Auslösend ist eine Relaxation (Entspannung) der glatten Gefäßmuskulatur. Sie wird durch viszeromotorische Fasern des vegetativen Nervensystems hervorgerufen. Verschiedene lokal gebildete Mediatoren, wie z.B. Bradykinin, Acetylcholin und Endothelin können durch eine Stimulation ihrer endothelialen Rezeptoren (z.B. B2-,M3-, oder ET-B-Rezeptor) unter anderem eine vermehrte Bildung von NO bewirken, wodurch entsprechend Vasodilatation bzw. Gefäßdilation verursacht wird. Auch eine erhöhte Expression endothelialer NO-Synthase und entsprechend induzierte NO-Bildung führt zum gleichen Effekt.

Stickstoffmonoxid (NO) selbst wird durch die Umwandlung von L-Arginin in NO über NO-Synthasen (NOS) in verschiedenen Teilen des Körpers produziert. Die endotheliale NOS (eNOS) produziert NO, das explizit als endogener Vasodilatator wirkt, in die Gefäßzellen diffundiert und die Entspannung der glatten Muskulatur durch Bindung an die Guanylylcyclase vermittelt. Die Guanylylzyklase wandelt GTP in cGMP um, das die cGMPabhängigen Proteinkinasen aktiviert. Diese Proteinkinasen phosphorylieren dann verschiedene lonenkanäle im endoplasmatischen Retikulum (ER), wodurch Kalzium sequestriert und eine Kalziummobilisierung innerhalb der Zelle verhindert wird. Infolgedessen entspannen sich die glatten Muskelzellen (Jennifer Witek; Anand D. Lakhkar., Treasure Island (FL): StatPearls Publishing; 2024 Jan). Die vorliegende Erfindung ist nicht auf diesen Mechanismus limitiert.

Die Induktion Endothel-abgeleiteter NO-abhängiger Vasodilatation ermöglicht die Vorbeugung und/oder Behandlung von Zuständen, Indikationen und/oder Erkrankungen, welche durch endothel-abgeleitete NO-abhängige Vasodilatation vermieden, verbessert bzw. therapiert werden können. Bevorzugt wird der zu behandelnde Zustand, die Indikation und/oder Erkrankung vor der Behandlung diagnostiziert.

Die erfindungsgemäß zu behandelnde Indikation ist bevorzugt ausgewählt aus der Gruppe bestehend aus Hautalterung, Hauterkrankungen, Atrophie, akute und/oder chronische Wunden und Dermatitis.

Beispielhafte Hauterkrankungen sind Akne, mikrobielle Hautinfektionen, Hautentzündungen, raue Haut, trockene Haut, Hautirritationen, Juckreiz, Pruritus, Allergien, Psoriasis, psoriatische Arthritis, Ekzeme, Scleroderma, Rosazea, systemischen Lupus erythematosus, Akne und Anfälligkeit gegenüber Kontaktallergien.

"Atrophie" beschreibt eine Verkümmerung bzw. Verkleinerung eines Gewebes, eines Organs oder einer einzelnen Zelle, die mit einer Minderung der Funktion einhergeht. Atrophie kann durch Volumen- bzw. Größenabnahme der Zellen oder durch Abnahme der Zellzahl charakterisiert sein.

Erfindungsgemäß ist Atrophie bevorzugt ausgewählt aus Altersatrophie, Inaktivitätsatrophie, trophoneurotischer/nervaler Atrophie, vaskulärer Atrophie, Druckatrophie, endokrinerAtrophie und/oder Atrophie der Haut.

"Altersatrophie" ist durch eine Volumenabnahme aller funktionalen (parenchymatösen) Zellen gekennzeichnet, was insbesondere im Gehirn und Herz sehr ausgeprägt sein kann.

"Inaktivitätsatrophie" wird durch fehlende Beanspruchung eines Organes, zum Beispiel bei einem eingegipsten Bein, induziert.

Bei Schädigung eines Nervs atrophiert der von ihm (nicht mehr) versorgte Muskel; es tritt "trophoneurotische/nervale Atrophie" ein.

Gelangen durch die Blutgefäße nicht genügend Sauerstoff und Nährstoffe zu den Zellen, können diese ebenfalls atrophieren. Dies kennzeichnet "vaskuläre Atrophie". Voraussetzung hierfür ist, dass die unzureichende Versorgung sich langsam entwickelt (zum Beispiel eine sich über Monate verengende Nierenarterie).

"Druckatrophie" entsteht beispielsweise beim Drücken von Tumoren auf umgebendes Gewebe, wodurch benachbarte Zellen atrophieren können.

"Endokrine Atrophie" kann aufgrund endokriner Signale, wie zum Beispiel bei Cortison-Therapie, auftreten.

Eine mögliche Nebenwirkung bei der Glucocorticoid-Behandlung des Atopischen Ekzems ist "Atrophie der Haut" ("Hautverdünnung").

Als "Dermatitis" wird hierin eine entzündliche Reaktion der Haut bezeichnet, die vornehmlich die Dermis (Lederhaut) erfasst. Der Begriff Dermatitis umfasst auch Ekzeme. Die zu behandelnde Dermatitis ist erfindungsgemäß bevorzugt ausgewählt aus Ekzemen, Badedermatitis, Dermatitis herpetiformis Duhring, exfoliativer Dermatitis, Dermatitis exfoliativa neonatorum, Dermatitis factitia, Dermatitis ulcerosa, perioraler Dermatitis, Stauungsdermatitis, Lichtdermatosen, Strahlendermatitis (insbesondere Dermatitis Solaris) atopische Dermatitis, Windeldermatitis, Kontaktdermatitis, Inkontinenz-assoziierte Dermatitis, Psoriasis und/oder Xerodermie.

Der Begriff "Ekzem" wie hierin verwendet umfasst eine Gruppe entzündlicher Hauterkrankungen, die sich in einer nicht-infektiösen Entzündungsreaktion der Haut äußern. Ekzeme können durch verschiedene Auslöser hervorgerufen werden. Sie sind durch eine typische Abfolge von Hautreaktionen charakterisiert (Hautrötung, Bläschenbildung, Nässen, Krustenbildung, Schuppung).

Bei "Badedermatitis" (Zerkariendermatitis) handelt es sich um eine Infektion durch Zerkarien verschiedener Saugwürmer.

"Dermatitis herpetiformis Duhring" ist eine chronisch-rezidivierende Autoimmunerkrankung mit subepidermaler Blasenbildung,
Bei "Dermatitis factitia" handelt es sich um eine artifizielle Störungaufgrund selbstschädigender Handlungen.

"Dermatitis ulcerosa" ist durch eine Vaskulitis mit einzelstehenden Ulzerationen gekennzeichnet.

"Periorale Dermatitis" (Syn. Rosacea-artige Dermatitis) beruht auf einer Entzündung der Gesichtshaut durch übermäßige Verwendung von Kosmetika.

"Stauungsdermatitis" ist in der Regel zu Beginn durch venöse Zirkulationsstörungen wie die Chronisch-venöse Insuffizienz bedingt.

"Atopische Dermatitis" ist eine chronische, nicht ansteckende Hautkrankheit, die zu den atopischen Erkrankungen gehört. Weitere geläufige Bezeichnungen sind Neurodermitis oder atopisches Ekzem.

"Psoriasis" oder Schuppenflechte oder Psoriasis ist eine nicht-ansteckende chronische Autoimmunkrankheit, die sich vor allem als entzündliche Hautkrankheit manifestiert. Nicht selten betrifft die Systemerkrankung auch andere Organe, insbesondere Gelenke und zugehörige Bänder sowie angrenzende Weichteile (Psoriasisarthritis), die Augen (Uveitis), das Gefäßsystem, das Herz sowie die Genitalien. Darüber hinaus kann sie mit Diabetes mellitus und Schlaganfällen einhergehen

Ebenfalls umfasst sind Krankheiten der Haut durch Strahleneinwirkung, wie Lichtdermatosen aufgrund übermäßiger Sonnenexposition (Sonnenbrand: Dermatitis solaris), photoallergischer oder photoxischer Reaktionen (z. B. Berloque-Dermatitis, Wiesengräserdermatitis) oder Strahlendermatitis (Radiodermatitis) als Folge ionisierender Strahlung.

Neben den beschriebenen bevorzugt zu behandelnden Indikationen können erfindungsgemäß eine Reihe weiterer Indikationen behandelt werden. Diese umfassen unter anderem Erkrankungen des Herzkreislaufsystems, wie beispielsweise chronische Myokardinsuffizienz, akute Rechtsherzinsuffizienz, koronare Herzkrankheit bzw. Angina pectoris, Hypertonie, insbesondere pulmonale Hypertonie und persistierende pulmonale Hypertonie bei Neugeborenen (PPHN), und Erkrankungen der Atemwege und/oder der Lunge wie beispielsweise akutes Atemnotsyndrom (ARDS), Bronchiolitis, insbesondere akute Bronchiolitis, Viruserkrankungen der Atemwege und/oder der Lunge, insbesondere COVID-19, Cor pulmonale,chronische Sinusitis, Sichelzellanämie, Erektile Dysfunktion, (benigne) Vergrößerung der Prostata und/oder Glaukom. Pulmonale Hypertonie kann insbesondere nach Herzoperationen auftreten.

Die hierin beschriebenen Zusammensetzungen können erfindungsgemäß auch zur Immunmodulation verwendet werden. NO verändert das Gleichgewicht von T-Helfer 1 (TH1) und T-Helfer 2 (TH2) Zellen. Insbesondere verringert NO die Proliferationsrate von TH1-Zellen und die Synthese des Zytokins IL-2, erhöht aber die Produktion von IL-4-Zytokinen aus TH2-Zellen. Auf diese Weise kann NO Entzündungsreaktionen auf virale und bakterielle Krankheitserreger hemmen. Außerdem wird angenommen, dass NO die Adhäsion und Rekrutierung von Leukozyten beeinflusst (Bogdan C. Nitric oxide and the immune response. Nat Immunol. 2001 Oct;2(10):907-16).

Im Rahmen der vorliegenden Erfindung kann jede geeignete Verabreichungsform gewählt werden. Bespielhafte Verabreichungsformen umfassen oral, intravenös, intramuskulär, kutan, subkutan, nasal, durch Inhalation und/oder rektal.

Die Verabreichung kann topische oder systemische Wirkung erzielen. Unter einer topischen Anwendung ist eine äußerliche Anwendung, insbesondere eine örtliche, äußerliche Anwendung zu verstehen.

Eine kutane und insbesondere topische Anwendung ist besonders bevorzugt.

Bei Erkrankungen der Atemwege und/oder der Lunge ist eine Verabreichung durch Inhalation bevorzugt.

Eine andere bevorzugte Verabreichung erfolgt transdermal, insbesondere bevorzugt durch Verwendung eines Pflasters. Transdermale Pflaster sind flexible und dünne pharmazeutische Zubereitungen, die auf die Haut aufgeklebt werden. Sie geben die enthaltenen Wirkstoffe kontinuierlich über die Hautbarriere in den Blutkreislauf ab. Entsprechend ist in dieser Ausführungsform das Erreichen eines systemischen Effekts bevorzugt.

Die erfindungsgemäß verwendete Zusammensetzung, insbesondere bei der topischen Verwendung, wird bevorzugt als Gel, Creme, Salbe, Lotion, Lösung, Suspension oder Milch angewendet.

Gele bestehen aus gelierten Flüssigkeiten und werden mit geeigneten Quellmitteln wie beispielsweise Celluose-Derivaten, Stärken, Carbomeren, Gelatine, Xanthan, Bentonit, Agar, Tragant, Carrageen, Alginate und/oder Pektin hergestellt.

Cremen sind halbfeste Zubereitungen, die in der Regel zur Anwendung auf der Haut oder Schleimhaut vorgesehen sind. Es handelt sich um mehrphasige Zubereitungen, die aus einer lipophilen und einer wässrigen Phase bestehen.

Salben sind halbfeste Zubereitungen zur äußerlichen Anwendung. Sie bestehen aus einer einphasigen Grundlage, in der feste oder flüssige Substanzen dispergiert sein können. Dies im Unterschied zu den Cremen, die mehrphasig sind und aus einer lipophilen und einer wässrigen Phase bestehen.

Lotionen sind Zubereitungen zur äußerlichen Anwendung auf der Haut mit einer flüssigen bis halbfesten Konsistenz.

Lösungen sind flüssige Zubereitungen, bei welchen die Wirk- und Hilfsstoffe in Wasser oder einem beliebigen anderen geeigneten Lösungsmittel (z.B. einem Öl) gelöst vorliegen. Lösungen können insbesondere bei der oralen Verabreichung kurz vor der Anwendung, beispielsweise aus einem Pulver, frisch hergestellt werden.

Suspensionen sind heterogene Stoffgemische, welche aus einer festen und flüssigen Phase bestehen. Die feste Phase kann sich mit der Zeit am Boden des Aufbewahrungsgefäßes absetzen. Deshalb sollten Suspensionen häufig direkt vor der Anwendung geschüttelt werden.

Der pH-Wert der topischen Zusammensetzung beträgt erfindungsgemäß bevorzugt, 3,0 bis 7,0, stärker bevorzugt 3,0 bis 5,9, noch stärker bevorzugt 3,5 bis 5,4 und besonders bevorzugt 4,0 bis 5,0 (gemessen bei 21 °C mittels pH-Meter, Mettler-Toledo SevenCompact S220). In diesem Bereich sind die erfindungsgemäßen Zusammensetzungen nicht nur chemisch, physikalisch und mikrobiologisch besonders stabil, sondern auch medizinisch und kosmetisch äußerst verträglich für eine topische Verabreichung. Besonders geeignete pH-Werte für die erfindungsgemäße Zusammensetzung sind: 3,5; 3,6; 3,7; 3,8; 3,9; 4,0; 4,1; 4,2; 4,3; 4,4; 4,5; 4,6; 4,7; 4,8; 4,9; 5,0; 5,1; 5,2; 5,3; und 5,4. Der pH-Wert der Zusammensetzung wird bevorzugt eingestellt mithilfe eines oder mehrerer pH-Modifizierungsmittel. Geeignete pH-Modifizierungsmittel können Säuren, Basen und/oder Puffersysteme sein, um den pH-Wert der Zusammensetzung zu stabilisieren oder zu beeinflussen. Typische pH-Modifizierungsmittel gemäß der vorliegenden Erfindung sind Adipin-, Zitronen-, Äpfel-, Bernstein-, Wein-, Ascorbin-, Phosphor-, Milch- und Fumarsäure sowie die entsprechenden Salze, sowie Natriumalginat, Polyacrylsäure, Natriumcarbonat und Natriumbicarbonat. Im Zusammenhang mit pH-Modifizierungsmitteln bezieht sich der Begriff Salz auf Alkalimetallsalze oder Erdalkalimetallsalze, sofern nichts anderes angegeben ist.

Die erfindungsgemäße Zusammensetzung enthält Dimethylglycin und/oder ein Salz von Dimethylglycin vorzugsweise in einem Anteil von 0,00001 Gew.-% bis 25,0 Gew.- %, bezogen auf das Gesamtgewicht der Zusammensetzung.

In einer bevorzugten Ausführungsform enthält die erfindungsgemäße Zusammensetzung Dimethylglycin und/oder ein Salz von Dimethylglycin in einem Anteil von 0,001 Gew.-% bis 10,0 Gew.-%, bevorzugter von 0,01 Gew.-% bis 8,0 Gew.-%, mehr bevorzugt von 0,1 Gew.-% bis 6,0 Gew.-%, noch mehr bevorzugt von 0,3 Gew.-% bis 5,0 Gew.-%, insbesondere von 0,5 Gew.-% bis 3,0 Gew.-%, jeweils bezogen das Gesamtgewicht der Zusammensetzung. In einer bevorzugten Ausführungsform der Erfindung kann die erfindungsgemäße Zusammensetzung 0,1 Gew.%, 0,2 Gew.%, 0,3 Gew.%, 0,4 Gew.%, 0,5 Gew.%, 0,6 Gew.%, 0,7 Gew.%, 0,8 Gew.%, 0,9 Gew.%, 1,0 Gew.%, 1,1 Gew.%, 1,2 Gew.%, 1,3 Gew.%, 1,4 Gew.%, 1,5 Gew.%, 2,0 Gew.% oder 2,5 Gew.% Dimethylglycin und/oder ein Salz von Dimethylglycin enthalten, jeweils bezogen auf das Gesamtgewicht der Zusammensetzung.

Bevorzugt enthält die erfindungsgemäße Zusammensetzung Dimethylglycin und/oder ein Salz von Dimethylglycin als chemischen Reinstoff, einschließlich der jeweiligen Solvate und Hydrate (z.B. des Dihydrats von Natriumdimethylglycinat), da so die Reinheit der Zusammensetzung erhöht und das Auftreten von unerwünschten Nebenwirkungen vermindert werden kann.

In einer bevorzugten Ausführungsform enthält die erfindungsgemäße Zusammensetzung wenigstens einen weiteren Wirkstoff. Der wenigstens ein weiterer Wirkstoff kann in Abhängigkeit von der zu behandelnden Indikation ausgewählt werden.

Bevorzugt ist dabei wenigstens ein Wirkstoff ausgewählt aus der Gruppe umfassend Antibiotika, Antimykotika (bei Superinfektion durch Pilze), Antihistaminika (symptomatisch bei Juckreiz), Glukokortikoide, Retinoide (bei Psoriasis, Akne, Rosazea), Immunsuppressiva, Menthol, Biotin, Zink PCA, Koffein, Niacinamid, Panthenol, Ectoin, Ubichinon-10, Taurin, Echinacea, Tocopherylacetat, NO-freisetzenden Mitteln und Kombinationen davon.

Antibiotika bezeichnen allgemein Arzneistoffe zur Behandlung bakterieller Infektionskrankheiten, unabhängig davon, ob sie hoch- oder niedermolekular, natürlicher oder synthetischer Herkunft sind.

Antimykotika sind antimikrobiellen Substanzen, die gegen durch Pilze verursachte Erkrankungen wirken.

Antihistaminika sind Wirkstoffe, die die Wirkung des körpereigenen Botenstoffs Histamin abschwächen oder aufheben, indem sie Histamin-Rezeptoren blockieren oder ihre Rezeptoraktivität noch unter die Basalaktivität absenken.

Glukokortikoide sind Hormone, insbesondere Steroidhormone. Die Glucocorticoide haben vielfältige physiologische und therapeutische Wirkungen. Sie beeinflussen den Stoffwechsel, den Wasser- und Elektrolythaushalt, das Herz-Kreislaufsystem und das Nervensystem. Ferner bewirken sie eine Entzündungshemmung und sind immunsuppressiv.

Retinoide sind Derivate von Vitamin A. Retinoide haben sebostatische, antiproliferative, proapoptotische und entzündungshemmende Eigenschaften.

Immunsuppressiva sind allgemein Substanzen, welche die Funktion des Immunsystems reduzieren.

Menthol ist ein monocyclischer Monoterpenalkohol und kann der erfindungsgemäßen Zusammensetzung als die Durchblutung stimulierender Wirkstoff zugesetzt werden. Zudem kann Menthol eine erfrischende sensorische Stimulierung der Haut bewirken.

Biotin, welches auch als Vitamin B7 oder Vitamin H bezeichnet wird, ist ein wasserlösliches Vitamin aus dem B-Komplex. Biotin kann die Haut stärken.

Zink PCA ist das Zinksalz des L-Pyrrolidon-Carboxylats und kann als Substanz mit antimikrobieller Wirkung der erfindungsgemäßen Zusammensetzung zugesetzt werden.

Koffein ist ein zur Klasse der Methylxanthine gehörendes Methylxanthinalkaloid. Es ist eine bittere kristalline Substanz, kann als Purinderivat betrachtet werden und ist chemisch mit den Adenin- und Guaninbasen der Desoxyribonukleinsäuren und der Ribonukleinsäure verwandt. Der IUPAC-Name von Koffein ist 1,3,7-Trimethyl-3,7-dihydro-1H-purin-2,6-dion. Koffein ist bekannt für verschiedene pharmakologische Effekte. So ist es insbesondere bekannt für seine stimulierende Wirkung auf das zentrale Nervensystem. Es existiert eine wachsende Anzahl von Nachweisen für einen positiven Einfluss auf eine Reihe von unterschiedlichen Dysfunktionen.

Niacinamid (auch Nicotinamid) ist das Amid der Nicotinsäure und wird auch als Vitamin B3 bezeichnet. Niacinamid führt zu einer Verringerung von oxidativem Stress.

Panthenol ist ein Provitamin, welches im Körper zu Pantothensäure (Vitamin B5) umgewandelt wird. Letzteres ist Teil des Coenzyms A und damit für den Hautstoffwechsel wichtig. Bei Einwirkung von Panthenol wird erfindungsgemäß die Hautelastizität und Feuchtigkeit weiter verbessert. Daneben werden Juckreiz und Entzündungen gelindert und die Wundheilung gefördert.

Ectoin ist eine zyklische Aminosäure und liegt in wässriger Lösung als mesomeriestabilisiertes Zwitterion vor. Es hat sich gezeigt, dass Ectoin vor UV-Strahlung schützt und bei der Behandlung entzündlicher Krankheiten hilfreich sein kann.

Ubichinon-10 (Q10 oder Coenzym Q10) ist ein Chinon-Derivat. Das zum Ubichinon-Pool gehörende Q10 gilt als Antioxidans und wirkt erfindungsgemäß stabilisierend auf die Haut.

Taurin oder 2-Aminoethansulfonsäure wirkt erfindungsgemäß als Antioxidans ebenfalls weiter stabilisierend auf die Haut.

Echinacea wirkt erfindungsgemäß beruhigend auf die Haut und lindert auftretenden Juckreiz sowie Spannungen. Zudem kann Echinacea die Blutzirkulation der Haut anregen.

Tocopherylacetat zeigt antioxidative Eigenschaften und wirkt erfindungsgemäß weiter stabilisierend auf die Haut.

Stickstoffmonoxid-freisetzende Mittel sind beispielsweise Nitroglycerin, Isosorbiddnitrat oder Molsidomin zur Behandlung der Angina pectoris - ein typisches Symptom der koronaren Herzkrankheit.

In einer bevorzugten Ausführungsform der Erfindung enthält die erfindungsgemäße Zusammensetzung mindestens einen weiteren Wirkstoff ausgewählt aus Menthol, Biotin, Zink PCA, Niacinamid, Panthenol, Ectoin, Koffein, Ubichinon, Taurin, Echinacea, Tocopherylacetat und Kombinationen davon jeweils in einem Anteil von 0,001 Gew.-% bis 10,0 Gew.-%, mehr bevorzugt 0,005 Gew.-% bis 7,50 Gew.-%, noch mehr bevorzugt von 0,01 Gew.-% bis 5,0 Gew.-%, insbesondere von 0,1 Gew.-% bis 3,0 Gew.-%, bezogen auf das Gesamtgewicht der Zusammensetzung.

Darüber hinaus kann die erfindungsgemäße Zusammensetzung wasserbasiert sein. Das bedeutet, dass sie beispielsweise 45,0 bis 85,0 Gew.-% Wasser enthält.

Die erfindungsgemäß eingesetzte Zusammensetzung kann wenigstens ein Additiv enthalten, welches bevorzugt ausgewählt ist aus der Gruppe umfassend Rückfetter, Konservierungsmittel, Stabilisatoren, Antioxidantien, Rheologiemodifikatoren, Verdickungsmitteln, Pflegemitteln, Farbstoffen, Lösungsmitteln und belibenen Kombinationen hiervon.

Rückfetter, auch Rückfettungsmittel oder Überfettungsmittel genannt, sind lipophile Substanzen, die eine störende Auswirkung auf die epidermale Barrierefunktion verhindern können. Beispiele für Rückfetter sind Wollwachs, Squalen, flüssiges Paraffin, pflanzliche Öle, Silikone und Cetylpalmitat.

Konservierungsmittel sind Substanzen, die zur Konservierung verwendet werden, indem sie die Zusammensetzung zersetzende Mikroorganismen abtöten und/oder deren Wachstum hemmen. Vorzugsweise können die Konservierungsmittel ausgewählt sein aus der Gruppe bestehend aus Benzoesäure, Benzoesäurederivaten, Sorbinsäure, Sorbinsäurederivaten, Salicylsäure, Salicylsäurederivaten, Phenoxyethanol, Parabenen und Kombinationen hiervon. In einer bevorzugten Ausführungsform werden Natriumbenzoat und/oder Kaliumsorbat als Konservierungsmittel in der erfindungsgemäßen Zusammensetzung eingesetzt. Natriumbenzoat setzt in leicht saurem Milieu Benzoesäure und Kaliumsorbat Sorbinsäure frei. Beiden Säuren wird eine antimikrobielle Wirkung zugeschrieben.

Stabilisatoren können lichtempfindliche Komponenten gegenüber Strahlung schützen und sind bevorzugt UV-Absorber wie beispielsweise Benzophenonderivate.

Die Zugabe von Duftstoffen kann für einen angenehmen Geruch der Zusammensetzung sorgen. Beispiele sind die dem Fachmann bekannten Parfums.

Ein Antioxidans oder Antioxidationsmittel ist eine chemische Verbindung, die eine Oxidation anderer Komponenten in der erfindungsgemäßen Zusammensetzung verlangsamt oder gänzlich verhindert. Als Antioxidantien kommen beispielsweise Zitronensäure, Ascorbinsäure und Butylhydroxyanisol in Frage.

Rheologiemodifikatoren und Verdickungsmittel können dabei helfen, die Applikationseigenschaften der erfindungsgemäßen Zusammensetzung zu verbessern. Als Rheologiemodifikator und Verdickungsmittel kann die Zugabe von Kochsalz (Natriumchlorid) in Betracht gezogen werden. Durch die Zugabe von Kochsalz kann die Fließfähigkeit der erfindungsgemäßen Zusammensetzung in gewissen Grenzen beeinflusst und auf das nötige Maß eingestellt werden. Als Verdicker können zusätzlich Cellulosederivate oder Polyacrylate eingesetzt werden.

Im Rahmen der Anmeldung sollen unter Pflegemitteln Substanzen verstanden werden, welche die Haut, insbesondere Kopfhaut pflegen. Hydrolysiertes Weizenprotein und Allantoin wirken pflegend auf Haut. Hydrolysiertes Weizenprotein hat hauptsächlich feuchtigkeitsspendende Eigenschaften.

Farbstoffe werden optional dazu verwendet, der erfindungsgemäßen Zusammensetzung eine charakteristische Farbe zu verleihen, so dass diese leicht von anderen Produkten unterschieden werden kann.

Als Lösungsmittel kann ein für den Fachmann auf diesem Gebiet gängiges Lösungsmittel oder Lösungsmittelgemisch eingesetzt werden. Bevorzugte Lösungsmittel sind Ethanol oder Butylenglykol, insbesondere 1,4-Butylenglykol, Propylenglykol und Isopropylalkohol. Bevorzugt ist Ethanol. Diese Lösungsmittel können vorzugsweise in einer Menge von 0,1 bis 70 Gew.% in der erfindungsgemäßen Zusammensetzung enthalten sein. Ihre Verwendung kann zu einem Frischegefühl führen und belastet durch das schnelle Abtrocknen das Frisurenbild in einem sehr geringen Maß. Außerdem helfen Lösungsmittel bei der Penetration der Wirkstoffe und verstärken somit deren Wirksamkeit.

Die Dosierung der erfindungsgemäß verwendeten Zusammensetzung ist von der zu behandelnden Indikation und dem zu behandelnden Patienten abhängig. Hier können u.a. Alter, Gewicht, Größe, Geschlecht, andere, insbesondere regelmäßig, eingenommene Medikamente, Schwangerschaft und/oder Vorerkrankungen eine Rolle spielen. Basierend auf derartigen Parametern kann die Dosierung vom Fachmann bestimmt werden. Die Zusammensetzungen können zur Behandlung und/oder Vorbeugung akuter und oder chronischer Zustände verabreicht werden. Insbesondere zur Behandlung chronischer Zustände kann eine Dauerbehandlung erfolgt. Beispielsweise kann chronische Myokardinsuffizienz durch die erfindungsgemäßen Zusammensetzungen allein oder in Kombination mit Herzglykosiden und/oder Diuretika als Dauerbehandlung erfolgen.

### Figuren

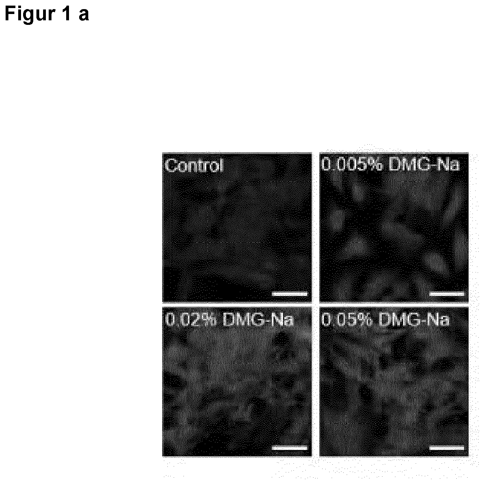

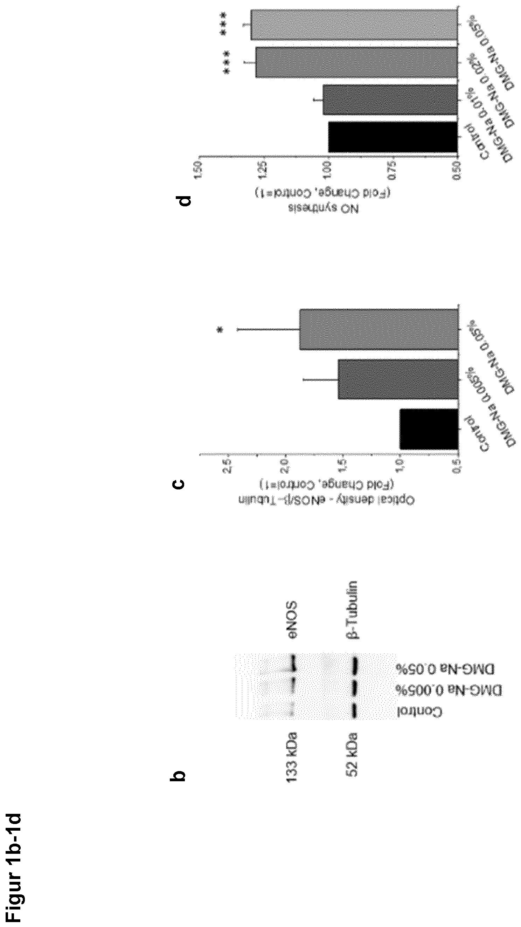

- **Figur 1:**: **Auswirkungen von DMG-Na auf verschiedene biologische Funktionen von HDMECs in vitro**
(**a**) Repräsentative Immunfluoreszenzmarkierung von eNOS (rot) in HDMECs, die mit verschiedenen Konzentrationen von DMG-Na behandelt wurden. Die Zellkerne wurden mit DAPI (blau) gegengefärbt. Skalenbalken = 50 µm. (**b**) Repräsentative Western-Blot-Immunolabeling von eNOS und β-Tubulin in HDMECs, die mit verschiedenen Konzentrationen von DMG-Na behandelt wurden. (**c**) Statistische Analyse der optischen Dichtemessungen an Immunoblots. Relative eNOS/β-Tubulin-Werte (Mittelwert ± SEM, n = 4 unabhängige Experimente) sind als x-fache Veränderung dargestellt, wobei sie auf die jeweiligen Kontrollen normalisiert wurden. Die P-Werte sind ebenfalls angegeben, wobei * den statistischen Unterschied im Vergleich zur Kontrolle (bestimmt durch Kruskal-Wallis mit Dunns Post-hoc-Test (* p<0,05)) markiert, (**d**) Statistische Analyse der mikrofluorimetrischen Messung von NO-Synthese. Die Werte (Mittelwert ± SEM, n=4 unabhängige Experimente) sind als x-fache Veränderung dargestellt, wobei sie auf die jeweiligen Kontrollen normalisiert wurden. P-Werte sind ebenfalls angegeben, wobei * den statistischen Unterschied im Vergleich zur Kontrolle mittels Einfaktorieller ANOVA mit Dunnetts Post-hoc-Test (*** p<0,001).

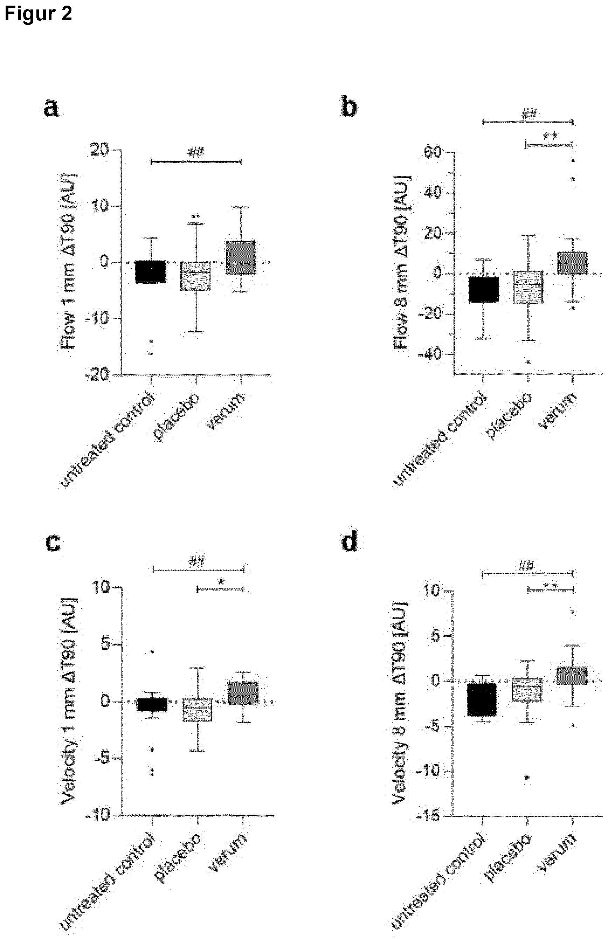

- **Figur 2:**: **Auswirkungen von DMG-Na auf die Mikrozirkulation menschlicher Haut in vivo**
(**a, b**) Dermale Mikrozirkulation, gemessen über den Blutfluss in 1 mm (**a**) und 8 mm (**b**) Hauttiefe nach den angegebenen Behandlungen (unbehandelte Kontrolle, Placebo oder Verum). (**c, d**) Dermale Mikrozirkulation gemessen anhand der Blutgeschwindigkeit in 1 mm (**c**) und 8 mm (**d**) Hauttiefe nach den angegebenen Behandlungen (unbehandelte Kontrolle, Placebo oder Verum). Box- und Whisker-Plots (Tukey-Methode), die den Median, Quartile und Extremwerte für die Unterschiede zwischen T90 und T0 (delta T90) des Blutflusses in willkürlichen Einheiten [AU] zeigen. Die mit dem Wilcoxon Signed Rank Test berechneten P-Werte sind wie folgt dargestellt: *p<0,05 und **p<0,01 signifikanter Unterschied im Vergleich zu Placebo; #p<0,05 und ##p<0,01 signifikanter Unterschied im Vergleich zur unbehandelten Kontrolle.

### 1. In vitro Zellkulturstudien

### Zellkultur

Adulte humane mikrovaskuläre Endothelzellen der Haut (HDMECs) wurden 2023 von Lonza Ltd (Basel, Schweiz) erworben und kultiviert in EBM^{™}-2 Endothelial Cell Growth Basal Medium, ergänzt mit EGM^{™}-2 MV Microvascular Endothelial SingleQuots^{™} Kit (beide von Lonza Ltd, Basel, Schweiz), enthaltend humanen epidermalen Wachstumsfaktor, vaskulären Endothelial Growth Factor, R³-Insulin-like Growth Factor-1, Ascorbinsäure, Hydrocortison, humanen Fibroblasten-Wachstumsfaktor-Beta, fötales Rinderserum und Gentamicin/Amphotericin-B und gemäß den Richtlinien des Herstellers kultiviert. Die HDMECs wurden von 2 verschiedenen weiblichen Spendern initiiert und es wurden die Passagen 4 bis 8 wurden für die Experimente verwendet.

### Immunzytochemie

HDMECs (5.000 Zellen pro 10 mm runde Glasdeckgläser), die mit DMG-Na (0,005%, 0,02% und 0,05%) behandelt wurden, wurden in 1% Paraformaldehyd (PFA, Merck KGaA, Darmstadt) für 10 Minuten bei Raumtemperatur fixiert und nach einem Waschschritt mit phosphatgepufferter Kochsalzlösung (PBS, Merck KGaA) für 5 Minuten mit -20 °C kalter Ethanol-Essigsäure (2:1) nachfixiert. Die Zellen wurden mit 0,25% Triton X-100 in PBS für 10 Minuten permeabilisiert, mit Antikörperverdünnungsmittel (Thermo Fisher Scientific, Waltham, MA) für 30 Minuten bei Raumtemperatur blockiert und dann mit primärem antihumanem eNOS Antikörper in einer Verdünnung von 1:100 (Abcam, Cambridge, UK; #ab76198) bei 4 °C über Nacht inkubiert. Nach dem entsprechenden Waschschritt mit PBS, wurden die Deckgläser zur Fluoreszenzfärbung mit sekundärem Alexa Flour-568 Ziege-Anti-Maus-lgG-Antikörper (Thermo Fisher Scientific, Waltham, MA; #A-11004) in einer Verdünnung von 1:500 bei Raumtemperatur für 45 Minuten inkubiert. Die Deckgläser wurden mit PBS gewaschen und die Zellkern-Gegenfärbung wurde mit 4',6-Diamidin-2-phenylindol (DAPI, Merck KGaA) für 1 Minute durchgeführt. Nach einem weiteren Waschschritt wurden die Zellen auf den Deckgläsern mit Fluoromount-G^{®} (SouthernBiotech, Birmingham, AL) eingedeckt. Die Bilder wurden mit einem konfokalen Nikon A1 Mikroskop (Nikon, Tokio, Japan) aufgenommen und mit der Software Image J Fiji, Version 2.9.0 (National Institutes of Health, Bethesda, MD) analysiert.

### Western Blot

HDMECs wurden in 6-Well-Platten ausgesät und für 24 Stunden mit DMG-Na (0,005% und 0,05%) behandelt. Nach der Behandlung wurden die Zellen in Lysispuffer (30 mM TRIS, pH 7,6, 140 mM NaCl, 5 mM EDTA, 50 mM NaF, 1 mM Na₃VO₄), der mit Proteaseinhibitoren ergänzt wurde, geerntet. Nach kräftigem Vortexen und Zentrifugieren wurde der Proteingehalt der Proben unter Verwendung des Pierce BCA Protein Assay Kits (Thermo Fisher Scientific, Waltham, MA) bestimmt. Gleiche Mengen an Proteinproben wurden einer 7,5%-igen SDS-PAGE unterzogen und auf Nitrocellulose-Membranen (Bio-Rad Laboratories, Hercules, CA) übertragen. Die proteinbindende Nitrocellulose-Membranen wurden mit 5% Magermilch in 1×TBST für 1 Stunde bei Raumtemperatur blockiert und dann mit primärem monoklonalem [M221] Anti-eNOS-Antikörper der Maus (Abcam, Cambridge, UK, #ab76198) in einer Verdünnung von 1:1000 in 2,5% Magermilch/1×TBST über Nacht bei 4 °C versetzt. Die Membranen wurden dreimal für 7 Minuten mit 1×TBST gewaschen und dann mit sekundärem Meerrettichperoxidase-markiertem Anti-Maus-lgG-Antikörper (GE Healthcare, Amersham, UK; #NA931V) in einer Verdünnung von 1:5000 in 1,5% Magermilch/1×TBST für 45 min bei Raumtemperatur inkubiert. Die Banden wurden mit SuperSignal West verstärkte Chemilumineszenz-Systeme (Thermo Fisher Scientific, Waltham, MA) unter Verwendung des CCD-Kamera-basierten Aufnahmegeräts Azure c300 Gel Bildgebungssystem (Azure Biosystems, Dublin, CA) visualisiert. Die densitometrische Analyse wurde unter Verwendung der Bildanalyse-Software Azure Spot Version 2.0.062 durchgeführt. Zur Beurteilung gleicher Beladung wurden die Membranen mit einem primären Anti-Beta-Tubulin-Antikörper (Thermo Fisher Scientific, Waltham, MA; #PA5-86071) in einer Verdünnung von 1:10.000 in 2,5% Magermilch/1×TBST neu versetzt und als sekundärer Antikörper Anti-Kaninchen-IgG-HRP (GE Healthcare, Amersham, UK; #NA934V) in einer Verdünnung von 1:10.000 in 1,5% Magermilch/1×TBST appliziert.

### Mikrofluorimetrische Messungen von NO

HDMECs wurden mit einer Dichte von 15.000 Zellen pro Well in einer schwarzen 96-WellPlatte mit klarem Boden (Greiner Bio-One, Kremsmünster, Österreich) ausgesät und in Wachstumsmedium bei 37 °C für 24 Stunden kultiviert. Anschließend wurden die Zellen mit 5 µM DAF-FM DA (4-Amino-5-methylamin-2',7'-difluororesceindiacetat) in phenolrotfreiem DMEM (beides von Thermo Fisher Scientific, Waltham, MA), ergänzt mit 0,5% FBS (Serana Europe GmbH, Brandenburg, Deutschland) für 1 Stunde bei 37 °C inkubiert. Die HDMECs wurden gewaschen, um den Überschuss der Probe zu entfernen, durch frisches Medium ersetzt und dann weitere 30 Minuten bei 37 °C inkubiert, um eine vollständige Entesterung der intrazellulären Diacetate zu ermöglichen. Nach der Inkubation wurden die Zellen mit DMG-Na (0,01%, 0,02% und 0,05%) behandelt und die Fluoreszenz wurde bei einer Anregung/Emission von 490/510 nm mit einem FlexStation II (Molecular Devices, Sunnyvale, CA) Fluoreszenz-Mikroplattenlesegerät gemessen (Nagy et al., 2003 und 2004).

### Statistische Analyse

Die Graphen wurden mit der Software OriginPro 8.6 (OriginLab Corporation, Northampton, MA) erstellt und die Daten mit GraphPad Prism Version 8.0.1. für Windows (GraphPad Software, San Diego, CA) analysiert. Bei normal verteilten Daten wurde eine einseitige ANOVA mit anschließendem Post-hoc Test nach Dunnett durchgeführt. Im Falle einer nicht normalen Datenverteilung wurde der Kruskal-Wallis-Test mit einem Mehrfachvergleichstest nach Dunn verwendet. Im Falle von intrazellulären Kalziummessungen wurden die Daten mit einer Zweifaktorielle ANOVA, gefolgt von einem Post-hoc Test nach Tukey, analysiert.

### 2. In-vivo-Studien am Menschen

### Studien design, Probanden

In dieser monozentrischen, einfach verblindeten, randomisierten, placebokontrollierten Studie zur Untersuchung der Wirksamkeit auf die Mikrozirkulation der Haut haben insgesamt 20 Probanden (16 Frauen und 4 Männer) im Alter von 18-30 Jahren (∅=24,90 Jahre) mit gesundem, normalem Hauttyp teilgenommen. Ausschlusskriterien waren eine Schwangerschaft und Stillzeit, eine Anwendung von topischen oder systemischen Behandlungen in den letzten Wochen, die die Beurteilung der Hautverträglichkeit des Studienprodukts beeinträchtigen könnten; Probanden, die sich innerhalb des letzten Monats einer Operation unter Vollnarkose unterzogen haben, eine übermäßige Exposition gegenüber Sonnenlicht oder UV-Strahlung innerhalb des letzten Monats und eine Teilnahme an einer anderen klinischen Studie während des Studienzeitraums, die mit der aktuellen Studie interferieren könnte. Außerdem wurden die Teilnehmer angewiesen, 24 Stunden vor dem Studienbeginn kein anderes ähnliches Produkt auf die Testflächen aufzutragen, keine Hormonbehandlung zu beginnen noch ihre übliche Hormonbehandlung zu ändern und nicht ihren Lebensstil wie Ernährung, Rauchen oder Sport zu ändern.

### Formulierungen

In der in-vivo-Studie am Menschen wurden die folgenden Gel-Formulierungen verwendet. Verum: N,N-Dimethylglycin-Natriumsalz (1%), Wasser, Poloxamer 407, Alkohol, Zitronensäure. Placebo: Natriumhydroxid, Wasser, Poloxamer 407, Alkohol, Zitronensäure. Beide Formulierungen enthielten die gleichen Konzentrationen an Inhaltsstoffen (außer DMG-Na und NaOH) und wurden auf pH 4,5 eingestellt.

### Wirksamkeitsstudie

Das O2C-System (LEA Medizintechnik GmbH, Gießen) wurde zur Messung der kurzfristigen Effekte des Wirkstoffs Natriumdimethylglycin (DMG-Na) auf den dermalen Blutfluss und der Geschwindigkeit zwischen 0 und 90 Minuten nach einer einmaligen Applikation in zwei Tiefen, 1 mm und 8 mm, verwendet. Die Messung des Blutflusses und der Geschwindigkeit (willkürliche Einheiten) wurde durch die Frequenz des Lichtes, das durch sich bewegende Erythrozyten verschoben wurde (Doppler-Effekt), bestimmt.

Die Probanden wurden gebeten, sich zu setzen. Der Unterarm blieb während des gesamten Versuchs in der gleichen Position. Die Probanden durften während des Versuchs in keiner Weise körperlich aktiv sein. Alle Messungen wurden nach einer Akklimatisierungszeit von 15 bis 20 min bei 22 °C unter den gleichen Bedingungen durchgeführt. Die Messungen wurden an der Innenfläche des Unterarms durchgeführt.

Zunächst wurden die Ausgangswerte am Unterarm vor der Anwendung des Verums und Placebos ermittelt. Anschließend wurde das Verum oder Placebo appliziert und der Kapillarblutfluss und die Kapillarblutgeschwindigkeit wurden 30 min, 60 min und 90 min nach der Applikation gemessen. Eine unbehandelte Fläche diente als Kontrolle. Die Testflächen auf der Innenseite des Unterarms wurden nach dem Zufallsprinzip ausgewählt (linker oder rechter Arm), wobei die Kontrolle immer am inneren Oberarm gemessen wurde, um die Messung von Auswirkungen des Verums auszuschließen. Die Daten der Mikrozirkulation wurden über 60 Sekunden (Messung im Sekundentakt) für jeden Messzeitpunkt aufgezeichnet.

### Statistische Auswertung

Die deskriptiven zusammenfassenden Statistiken wurden für die beiden Parameter getrennt für drei Gruppen und für alle vier Zeitpunkte berechnet. Die Statistiken umfassen Mittelwert und Medianwert, Standardabweichung, Minimum und Maximum. Zusätzlich wurden die individuellen Unterschiede zum Ausgangswert (Prä-Post-Differenz) ermittelt, um die Entwicklung über die Zeit zu erfassen, und als zusammenfassende Statistiken dargestellt.

Um den Behandlungseffekt zu untersuchen, wurden Gruppenvergleiche für Placebo vs. tatsächliche Behandlung und unbehandelte Kontrolle vs. tatsächliche Behandlung durchgeführt, wobei der nichtparametrische Wilcoxon Signed Rank Test auf einem Alpha-Niveau von 5% zweiseitig angewandt wurde, um die Prä-Post-Unterschiede zum Ausgangswert zu analysieren. Die Analyse wurde auf die beobachteten Werten angewandt, d.h. unter Berücksichtigung der individuellen Unterschiede von dem Ausgangswert für alle Berechnungen. Alle Unterschiede wurden bei p<0,05 als signifikant angesehen. Alle statistischen Analysen wurden mit SAS (SAS Institute, Cary, NC) Version 9.3 durchgeführt. Die Graphen wurden mit Prism Version 7.05 (GraphPad Software, San Diego, CA) erstellt.

### 3. Zusammenfassung

In der aktuellen Studie wurden die Auswirkungen von DMG auf die Mikrozirkulation und die Endothelfunktionen der menschlichen Haut untersucht.

In den durchgeführten *in-vitro*-Studien wurde überraschenderweise gefunden, dass DMG-Na die Expression von endothelialer Stickstoffmonoxid-Synthase erhöht und die Produktion von Stickstoffmonoxid (NO) induziert.

Darüber hinaus wurde in einer monozentrischen, einfach verblindeten, randomisierten, Placebo-kontrollierten *in-vivo-Studie* am Menschen überraschenderweise nachgewiesen, dass topisch appliziertes 1%-iges DMG-Na-Gel statistisch signifikant die dermale Durchblutung und die dermale Durchblutungsgeschwindigkeit im Vergleich zum Placebo und/oder der unbehandelten Kontrolle erhöht.

Diese Daten stellen zum ersten Mal den Nachweis im Menschen dar, dass topisches DMG die Intensität der dermalen Mikrozirkulation durch Eindringen in tiefere Hautschichten erhöht, was auf seine Wirkung auf HDMECs zurückgeführt werden kann, wo es eine vom Endothel abgeleitete, NO-abhängige Vasodilatation induziert.

### Im Detail

Im Rahmen der Experimente wurde untersucht, ob DMG-Na die NO-Freisetzung aus dem Endothel induzieren kann. Im ersten Teil der Studie wurden daher verschiedene zelluläre und molekulare Techniken eingesetzt und die Auswirkungen von DMG-Na *in vitro* auf primäre HDMECs gemessen.

Zunächst wurde untersucht, ob DMG-Na die eNOS-Expression von HDMECs beeinflusst. Wie gezeigt durch semiquantitative Immunozytochemie, schien DMG-Na die Expression von eNOS nach 24-stündiger Behandlung zu erhöhen (Figur 1a). Dies wurde quantitativ durch Western Blotting verifiziert, was darauf hindeutet, dass DMG-Na tatsächlich dosisabhängig und im Fall von 0,05% DMG-Na, statistisch signifikant (p=0,0189) den Proteingehalt von eNOS im Vergleich zur Kontrolle erhöht (Figuren 1b und 1c).

Schließlich wurde der Effekt von DMG-NA auf die NO-Synthese der HDMECs untersucht. Unter Verwendung von DAF-FM-DA-basierter Mikrofluorimetrie wurde überraschenderweise gefunden, dass 0.02% und 0.05% DMG-NA statistisch signifikant (p=0,0002 bzw. p=0,0001) die zelluläre NO-Produktion im Vergleich zur Kontrolle erhöht (Figur 1d).

Diese *in-vitro-*Daten zeigten eindeutig, dass DMG-Na auf die eNOS-Aktivierung und NO-Synthese fördert, was wiederum eine endothelabhängige Vasodilatation induzieren kann und die Mikrozirkulation der Haut, z.B. bei topischer Anwendung, verbessern kann.

Um diese Hypothese zu überprüfen, wurde eine monozentrische, einfach blinde, randomisierte, Placebo-kontrollierte *in-vivo*-Studie am Menschen entwickelt, in der die Auswirkungen eines topisch applizierten 1%-igen DMG-Na-Gels auf die Mikrozirkulationsparameter, den dermalen Blutfluss (das Volumen der Blutbewegung über die Zeit) und die dermale Blutflussgeschwindigkeit in der Haut (die Rate der Blutbewegung über eine Strecke), untersucht wurde.

Wie mit dem auf dem Doppler-Effekt basierenden O2C-System gemessen, erhöhte bei 1 mm Hauttiefe DMG-Na bereits bei T30 min nach topischer Applikation leicht den Blutfluss, was zu einer statistisch signifikanten Erhöhung der Flussrate bei T60 min (p=0,0073) und T90 min (p=0,0027) im Vergleich zur unbehandelten Kontrollfläche führte (Figur 2a und Tabelle 1). Bei 8 mm Hauttiefe war der Effekt von DMG-Na noch stärker. Es wurde eine statistisch signifikante Erhöhung des Blutflusses bei T90 min (p=0,0083) nach der Behandlung im Vergleich zu der mit Placebo behandelten Testfläche beobachtet, während im Vergleich zu der unbehandelten Kontrollfläche statistische Signifikanz bei allen Zeitpunkten (T30 min: p=0,0014, T60 min: p=0,0032, T90 min: p=0,0002) zu Gunsten der mit DMG-Na-behandelten Testfläche erreicht wurde (Figur 2)).

Neben der Blutflussrate erhöhte das topisch applizierte 1%-ige DMG-Na auch die dermale Durchblutungsgeschwindigkeit in beiden gemessenen Hauttiefen. Bei 1 mm wurde eine statistische Signifikanz bei T90 min (p=0,0266) im Vergleich zur Placebo-getesteten Fläche erreicht, während im Vergleich zur unbehandelten Kontrolle statistische Signifikanz bei T60 min (p=0,0215) und T90 min (p=0,0023) beobachtet wurde (Figur 2c und Tabelle 3). Ferner war in 8 mm Hauttiefe der geschwindigkeitserhöhende Effekt einer topischen DMG-Na-Applikation statistisch signifikant bei T30 min (p=0,0362) und T90 min (p=0,0042) im Vergleich zum Placebo, während im Vergleich zur unbehandelten Fläche die statistische Signifikanz zu allen Zeitpunkten (T30 min: p=0,0289, T60 min: p=0,0152, T90 min: p=0,0024) erreicht wurde (Figur 2d und Tabelle 4).

Bemerkenswert ist, dass im Verlauf dieser *in-vivo*-Studie eine Zunahme der Mikrozirkulation bei allen Probanden beobachtet wurde (ein Proband zeigte sogar eine leichte temporäre rote Fleckigkeit der Haut etwa 60 min nach Applikation des Verum-Produkts), was mit der Erhöhung der Mikrozirkulation korreliert. Es wurden jedoch keine unerwünschten Effekte wie z.B. Juckreiz, Brennen, Spannungsgefühl oder jegliche andere Beschwerden beobachtet.

Zusammengefasst liefern die Daten den ersten *in-vivo*-Nachweis im Menschen, dass topisches DMG die Intensität des dermalen Blutflusses durch Eindringen in die tieferen Hautschichten stark erhöht. Ferner zeigen die *in-vitro*-Experimente eindeutig, dass die oben genannten Effekte von DMG höchstwahrscheinlich auf seine Wirkung auf HDMECs zurückzuführen sind, was wiederum eine Endothel-abgeleitete, NO-abhängige Gefäßerweiterung induziert.

Die *in-vivo-* und *in-vitro*-Ergebnisse zeigen, dass DMG und insbesondere DMG-Na ein neuer Wirkstoff ist, z.B. bei Hautalterung, Atrophie, akuten oder chronischen Wunden, verschiedenen Dermatitis, bei denen die positiven Wirkungen von DMG, die epidermale Proliferation, Regeneration und Reparatur fördern, den dermalen Blutfluss und Geschwindigkeit erhöhen und/oder Schutzfunktionen auszuüben.

**Tabelle 1. Dermaler Blutfluss zwischen 0 und 90 min nach Gabe von Verum, Placebo und unbehandelter Kontrolle bei 1 mm Hauttiefe.**

| | Fluss [1 mm] | | | | Vorher-Nachher-Differenz im Fluss [1 mm] | | |
|---|---|---|---|---|---|---|---|
| | 0 min | 30 min | 60 min | 90 min | ΔT30 | ΔT60 | ΔT90 |
| Verum | 6.8±2.72 | 7.18±7.51 | 8.48±6.67 | 7.85±4.12 | 0.39±7.48 | 1.68±6.77^{##} | 1.05±4.16^{##} |
| Placebo | 8.58±3.60 | 7.99±7.43 | 7.20±4.85 | 6.91±3.59 | -0.59±8.15 | -1.38±5.23 | -1.67±5.17 |
| Unbehandelte Kontrolle | 9.87±6.75 | 6.06±2.40 | 6.44±3.34 | 7.54±5.48 | -3.80±5.47 | -3.42±5.39 | -2.32±4.81 |

| | | | | | | | |
|---|---|---|---|---|---|---|---|
| Die Werte sind Mittelwerte ± SD (n = 20). *p<0,05 und **p<0,01 signifikanter Unterschied im Vergleich zum Placebo; #p<0,05 und ##p<0,01 signifikanter Unterschied im Vergleich zur unbehandelten Kontrolle. | | | | | | | |

**Tabelle 2. Dermaler Blutfluss zwischen 0 und 90 min nach Gabe von Verum, Placebo und unbehandelter Kontrolle bei 8 mm Hauttiefe.**

| | Fluss [8 mm] | | | | Vorher-Nachher-Differenz im Fluss [8 mm] | | |
|---|---|---|---|---|---|---|---|
| | 0 min | 30 min | 60 min | 90 min | ΔT30 | ΔT60 | ΔT90 |
| Verum | 32.96±11.52 | 39.83±29.76 | 34.74±14.58 | 40.47 ±20.66 | 6.86 ±28.58^{##} | 1.77 ±13.16^{##} | 7.51 ±16.86**^{###} |
| Placebo | 43.58±18.92 | 39.85±18.92 | 39.60±21.82 | 36.89 ±20.46 | -3.73±14.09 | -3.98 ±12.15 | -6.69 ±14.55 |
| Unbehandelte Kontrolle | 44.25±14.83 | 34.78±11.71 | 35.34±12.17 | 34.91 ±13.14 | -9.47±10.69 | -8.91 ±10.31 | -9.34 ±9.90 |

| | | | | | | | |
|---|---|---|---|---|---|---|---|
| Die Werte sind Mittelwerte ± SD (n = 20). *p<0,05 und **p<0,01 signifikanter Unterschied im Vergleich zum Placebo; #p<0,05 und ##p<0,01, ###p<0.001 signifikanter Unterschied im Vergleich zur unbehandelten Kontrolle. | | | | | | | |

**Tabelle 3. Dermale Blutgeschwindigkeit zwischen 0 und 90 min nach Gabe von Verum, Placebo und unbehandelter Kontrolle bei 1 mm Hauttiefe.**

| | Fluss [8 mm] | | | | Vorher-Nachher-Differenz im Fluss [8 mm] | | |
|---|---|---|---|---|---|---|---|
| | 0 min | 30 min | 60 min | 90 min | ΔT30 | ΔT60 | ΔT90 |
| Verum | 10.53±1.26 | 11.52±5.98 | 11.47±5.09 | 11.07 ±1.63 | 0.99±5.5 | 0.95±4.57^{#} | 0.54 ±1.30*^{##} |
| Placebo | 11.02±1.82 | 11.72±6.01 | 10.74±3.65 | 10.44 ±2.22 | 0.70±6.36 | -0.28±2.58 | -0.58±1.91 |
| Unbehandelte Kontrolle | 11.69±3.81 | 10.10±1.93 | 10.25±2.07 | 10.94 ±2.26 | -1,59±2.84 | -1.44±2.77 | -0.75±2.32 |

| | | | | | | | |
|---|---|---|---|---|---|---|---|
| Die Werte sind Mittelwerte ± SD (n = 20). *p<0,05 und **p<0,01 signifikanter Unterschied im Vergleich zum Placebo; #p<0,05 und ##p<0,01 signifikanter Unterschied im Vergleich zur unbehandelten Kontrolle. | | | | | | | |

**Tabelle 4. Dermale Blutgeschwindigkeit zwischen 0 und 90 min nach Gabe von Verum, Placebo und unbehandelter Kontrolle bei 8 mm Hauttiefe.**

| | Fluss [8 mm] | | | | Vorher-Nachher-Differenz im Fluss [8 mm] | | |
|---|---|---|---|---|---|---|---|
| | 0 min | 30 min | 60 min | 90 min | ΔT30 | ΔT60 | ΔT90 |
| Verum | 13.75±2.04 | 14.89±4.45 | 13.94±2.55 | 14.46 ±2.81 | 1.15±4.92*^{#} | 0.20±2.53^{#} | 0.72 ±2.42**^{##} |
| Placebo | 14.74±3.64 | 13.63±3.35 | 13.77±4.02 | 13.41 ±3.56 | -1.11±2.79 | -0.97±2.85 | -1.33±2.76 |
| Unbehandelte Kontrolle | 15.47±3.58 | 13.64±2.61 | 13.48±2.62 | 13.71 ±2.81 | -1.84±2.05 | -2.00±1.66 | -1.76±1.75 |

| | | | | | | | |
|---|---|---|---|---|---|---|---|
| Die Werte sind Mittelwerte ± SD (n = 20). *p<0,05 und **p<0,01 signifikanter Unterschied im Vergleich zum Placebo; #p<0,05 und ##p<0,01 signifikanter Unterschied im Vergleich zur unbehandelten Kontrolle. | | | | | | | |

## Patentansprüche

1. Verwendung einer Zusammensetzung umfassend N,N-Dimethylglycin (DMG) und/oder ein Salz davon zur Induktion von Endothel-abgeleiteter NO-abhängiger Vasodilatation.

2. Verwendung nach Anspruch 1,
zur Behandlung einer Indikation, die ausgewählt ist aus der Gruppe bestehend aus Hautalterung, Hauterkrankungen, Atrophie, akute und/oder chronische Wunden und Dermatitis.

3. Verwendung nach Anspruch 4,
wobei die Atrophie ausgewählt ist aus Altersatrophie, Inaktivitätsatrophie, trophoneurotischer/nervaler Atrophie, vaskulärer Atrophie, Druckatrophie, endokrinerAtrophie und/oder Atrophie der Haut.

4. Verwendung nach Anspruch 4,
wobei die Dermatitis ausgewählt ist aus Badedermatitis, Dermatitis herpetiformis Duhring, exfoliativer Dermatitis, Dermatitis exfoliativa neonatorum, Dermatitis factitia, Dermatitis ulcerosa, perioraler Dermatitis, Stauungsdermatitis, Lichtdermatosen, atopische Dermatitis, Windeldermatitis, Kontaktdermatitis, Inkontinenz-assoziierte Dermatitis, Strahlendermatitis, Psoriasis und/oder Xerodermie.

5. Verwendung nach einem der vorhergehenden Ansprüche,
wobei die Zusammensetzung ein Salz von DMG enthält und insbesondere das Natrium-Salz von DMG (DMG-Na) ist.

6. Verwendung nach einem der vorhergehenden Ansprüche,
wobei die Zusammensetzung zur topischen Anwendung ist.

7. Verwendung nach einem der vorhergehenden Ansprüche,
wobei die Zusammensetzung ein Gel, Creme, Salbe, Lotion, Lösung, Suspension oder Milch ist.

8. Verwendung nach einem der vorhergehenden Ansprüche,
wobei die Zusammensetzung mittels eines Pflasters verabreicht wird.

9. Verwendung nach einem der vorhergehenden Ansprüche,
wobei die Zusammensetzung einen pH-Wert im Bereich von 3,0 bis 7,0, insbesondere 4,5 -5,0, aufweist.

10. Verwendung nach einem der vorgehenden Ansprüche,
wobei die Zusammensetzung 0,00001 bis 25,0 Gew.%, insbesondere 0,1 bis 10,0 Gew.%, N,N-Dimethylglycin (DMG) und/oder ein Salz davon enthält.

11. Verwendung nach einem der vorgehenden Ansprüche,
wobei die Zusammensetzung wenigstens einen weiteren Wirkstoff enthält.

12. Verwendung nach Anspruch 13,
wobei der wenigstens eine Wirkstoff ausgewählt ist aus Antibiotika, Antimykotika, Antihistaminika, Glukokortikoide, Retinoide, Immunsuppressiva, Menthol, Biotin, Zink PCA, Koffein, Niacinamid, Panthenol, Ectoin, Ubichinon-10; Taurin, Echinacea, Tocopherylacetat und Kombinationen davon.

13. Verwendung nach einem der vorhergehenden Ansprüche,
wobei die Zusammensetzung wenigstens ein Additiv enthält, ausgewählt aus Rückfettern, Konservierungsmitteln, Stabilisatoren, Antioxidantien, Rheologiemodifikatoren, Verdickungsmitteln, Pflegemitteln, Farbstoffen, Lösungsmitteln und Kombinationen hiervon.
